# EUROPEAN PATENT SPECIFICATION

(11) **EP 2 349 477 B1**
(45) Date of publication and mention of the grant of the patent: **18.07.2012**
(21) Application number: 08875215.9
(22) Date of filing: 22.10.2008
(51) Int. Cl.: A61N 5/06

(54) **SCALP LIGHT TREATMENT DEVICE FOR THE STIMULATION OF HAIR GROWTH**
KOPFHAUT-LICHTBEHANDLUNGSVORRICHTUNG ZUR STIMULIERUNG DES HAARWUCHSES
DISPOSITIF DE TRAITEMENT DU CUIR CHEVELU PAR LA LUMIÈRE, DESTINÉ À STIMULER LA CROISSANCE CAPILLAIRE

(43) Date of publication of application: 03.08.2011
(73) Proprietor: Unilever PLC, London, Greater London EC4Y 0DY (GB); Unilever N.V., 3013 AL Rotterdam (NL)
(72) Inventor: BIRMINGHAM, John, Joseph, Wirral Merseyside CH63 3JW (GB); BURRY, Jason, Shaun, Wirral Merseyside CH63 3JW (GB); HOPKINSON, Ian, Wirral Merseyside CH63 3JW (GB); TURNER, Graham, Andrew, Wirral Merseyside CH63 3JW (GB)
(74) Representative: Newbould, Frazer Anthony
(86) International application number: PCT/EP2008/064305
(87) International publication number: WO 2010/045973

(56) References cited:
- US-A- 2 397 757
- US-A- 5 300 097
- US-A1- 2008 172 112
- US-A1- 2008 172 113
- US-A1- 2008 172 115
- US-A1- 2008 177 255
- US-A1- 2008 215 123

## Description

The present invention relates to a scalp treatment device, and more particularly to a hand held scalp treatment device using light for the stimulation of hair growth.

### Background and Prior Art

Light biostimulation has been recognized as a method for providing stimulation to the scalp which has beneficial and therapeutic effects in terms of stimulating the natural growth of hair. For example, low level laser light is believed to enhance the physiological state of the scalp and encourage hair growth when radiated onto the hair follicles on the scalp. Non-coherent light sources such as light emitting diodes (LED) have also been described in this context.

A number of hand held devices have been designed to exploit the biostimulation effects of light sources such as low level laser and LED in a convenient and portable format.

WO01/60457 describes a hand-held, comb-like device emitting laser beams from laser diodes set in a row centred between two parallel rows of teeth for parting hair. The parting of the hair by the comb teeth is said to improve contact between the laser beam or beams and the scalp to be treated.

WO02/102228 describes a similar device, further including a stepped beam splitting reflector which splits one or more laser beams to allow a laser to simultaneously provide multiple laser beams which are distributed across a segment of an individual's scalp. The beam splitting reflector is mechanically aligned with the laser source and has a zigzag structure that mechanically deflects portions of the laser beam as it passes over the peaks of the beam splitting reflector. The portions of the laser beam form a line of reflected laser beams that project towards the users scalp.

In use, the devices described in WO01/60457 and WO02/102228 emit a narrow strip of parallel light beams from the comb housing onto the scalp. This produces a narrow strip of small, discrete beam spots on the surface under treatment. A problem associated with this pattern of scalp irradiation is that a proportion of the hair follicles present in the surface under treatment may be inadequately irradiated, or not irradiated at all, due to the size and spacing of the beam spots when applied to the scalp. This proportion may be especially significant in areas where the density of hair follicles is high. Repeated passage of the device over the area in question is not a satisfactory solution since there is an overall limit to the amount of laser power that can be applied to a person's scalp before the benefits of laser treatment are outweighed by more harmful conditions such as scalp redness, dryness and peeling.

The object of the present invention is to improve the pattern of scalp irradiation delivered from a hand held scalp treatment device using light for the stimulation of hair growth.

The invention solves the problem of uneven or inadequate scalp irradiation by means of a configuration which channels light through a plurality of hollow tines and spreads the light outwardly from the ends of the tines onto the surface of the scalp to be treated.

US 5,303,722 describes a light comb including a plurality of comb teeth, a device for guiding light from a light source through the comb back into the comb teeth, and a device for radiating the light from the comb teeth. However, the purpose of the device of US 5,303,722 is to selectively irradiate hair to be bleached which is located between the teeth of the comb, and not the scalp. Concave mirrors are used in the tips of the comb teeth to guide light away from the scalp and into the intermediate spaces between the comb teeth. Documents US 5,300,097, US 2008/172112, US 2008/172113,

US 2008/172115, US 2008/177255 disclose the preamble of claim 1.

### Summary of the Invention

The present invention provides a hand held hair treatment device for the stimulation of hair growth on the scalp, as defined in claim 1.

The handle portion may be any structure which facilitates gripping of the device by the user. For example, the handle portion may be formed by an elongated section of the housing, or alternatively may be a strap provided on the housing so that the device may be retained by the hand of the user.

The hollow tines serve to part the hair, enabling light to reach the surface of the scalp. The tines also provide the structures through which light is channelled by the light guide means. The longitudinal lengths of the tines may be formed from any material which enables the tines to perform these functions. The longitudinal lengths of the tines are formed from a substantially rigid material such as polypropylene, polyethylene, polyester, polycarbonate, polyvinylchloride or other material having similar characteristics.

The individual tines may have a longitudinal length ranging from 0.5 to 5cm, preferably about 1 to 2cm.

The individual tines may have a diameter ranging from 0.5 to 5mm, preferably about 1 to 2mm.

The tip sections of the tines are formed from a soft and flexible material (such as thermoplastic elastomer, rubber or other material having similar characteristics). This provides a pleasant feel when combing the user's hair or massaging the user's scalp. To this end, it is preferable to round the ends of the tip sections of the tines.

The tines are preferably arranged in a rectangular array of 1 to 3, preferably 2 to 3 rows.

The device may also include further tines in addition to those described above, which do not contain light. Such non-light containing tines may serve to assist in hair parting, scalp exposure, scalp massage or the like.

The device comprises a light source disposed within the housing and configured to output light. Suitable light sources include low power lasers, light emitting diodes or low power semiconductor lasers, capable of delivering light from the visible or infrared spectrum. Examples of preferred light sources are low power lasers having a wavelength ranging from about 600nm to about 1000nm, and an intensity ranging from about 1mW/cm² to about 150mW/cm². Such lasers are available commercially as complete modules which are small cylindrical units in which is encased the lasing medium which creates the laser beam and the electronics that control and process the electric current that passes through the lasing medium. Other preferred light sources are light emitting diodes having a wavelength ranging from about 620 to 690 nm, and an intensity ranging from about 1mW/cm² to about 150mW/cm².

The light source may deliver continuous or pulsed light, and may be powered by mains, battery or both. Battery power is advantageous for portability of the device. The battery can be rechargeable or disposable, preferably rechargeable.

Combinations, pluralities or assemblies of any of the above described light sources may also be used. For example, a plurality of laser modules as described above may be disposed in the housing at the proximal ends of the tines.

The light guide means serve to channel light from the housing and through the tines. Preferably the light guide means is constituted by a system of optical fibres which extends from the light source or sources, into the tines and through the tines. Suitable materials used to form the optical fibres may include fused silica, glass or an optical polymer such as polymethylmethacrylate. The optical fibres may extend singly through individual tines or may be provided in bundles.

The optical devices are configured to spread light incident upon them outwardly from the distal ends of the tines onto the surface of the scalp to be treated. By 'outwardly' is meant that the light beam is projected onto the scalp such that it passes outside the footprint of the tine on the scalp. In other words the optical modifier diffuses the light from the general axis of the tine. This improves the coverage of the light on the scalp's surface and reduces the chances of the user burning the scalp by repeatedly combing over the same area.

The optical devices will typically include one or more optical components which are capable of spreading light incident upon them in a controlled manner in the desired direction(s), so that a light beam footprint of a predetermined shape is produced on the target surface. By "light beam footprint" is meant the area of the target surface irradiated by the light beam at any moment, or in other words, the intersection of the light beam with the target surface.

Examples of preferred optical components include lenses, optical fibres, mirrors, transparent materials, semi-transparent materials, prisms, reflective coatings, reflecting grooves, beam splitters, light channels or gratings.

Most preferably, the optical modifier is a lens.

Combinations, pluralities or assemblies of any of the above described optical components may also be used.

The optical devices are located at the distal ends of the tines. In order to optimize the pattern of scalp irradiation delivered from the device, it is preferred that the optical devices are located slightly upstream from the actual end points of the tines. This slight spacing ensures that the light which is spread from the distal ends of the tines by the optical devices is not "buried" in the soft scalp surface when the tines are applied to the scalp.

Accordingly, in a preferred tine construction, the optical devices are disposed in a region adjacent to the tip sections of the tines, in the direction of the head portion of the device.

The optical modifier is disposed from 2 to 10 mm from the distal end of the tine, more preferably from 4 to 6 mm.

An especially preferred tine construction has a substantially rigid longitudinal length and a soft flexible tip section, with the optical device located at the interface between these two component parts.

The optical devices are configured to spread light outwardly from the distal ends of the tines onto the surface of the scalp to be treated. The optical devices serve to deliver an optimal pattern of irradiation to the target scalp surface. By "optimal pattern of irradiation" is generally meant a pattern in which the irradiation is spread over the target surface so that it covers at least 60%, preferably at least 80% of the total area of that target surface. Preferred patterns of irradiation have a regular or substantially regular spatial distribution.

In a preferred construction, the optical devices of the tines are configured so that they produce an array of circular light beam footprints on the target scalp surface. It is preferable to avoid a significant degree of overlap between individual footprints in the array. Accordingly, it is preferred that each individual footprint borders on those footprints which are adjacent to it in the array. The individual beam footprints could alternatively be square, polygonal, or any other shape suitable to deliver an optimal pattern of irradiation.

### Brief Description of the Drawings

Embodiments of the present invention will now be described with reference to the following non-limiting drawings in which:
Figure 1 depicts a partial schematic sectional view of a hair treatment device of the invention applied to the surface of the scalp;
Figure 2 depicts a partial schematic top planar view of the device of Figure 1 in use, illustrating the pattern of light beam footprints produced by the device, and
Figure 3 depicts an enlarged schematic sectional view of the distal end of a tine of the device of Figure 1 in use on the surface of the scalp.

### Detailed Description of the Invention

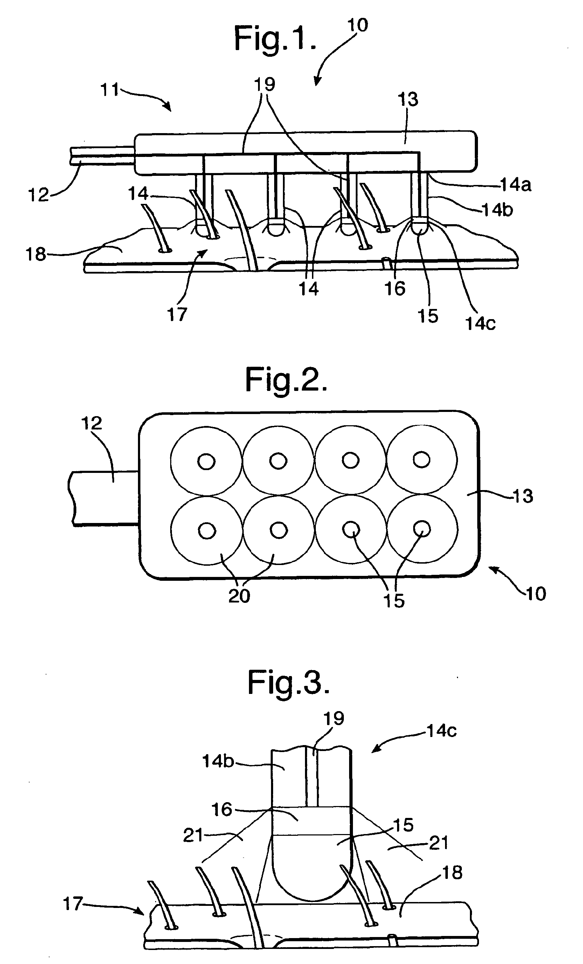

As shown in Figures 1 to 3, a hair treatment device **10** has a housing **11** formed from a handle portion **12** (shown in part) which is connected to a head portion **13**. Two parallel rows of four hollow tines **14** are connected to head portion **13** and project downward from head portion **13**. Each individual tine **14** has a proximal end **14a** affixed to head portion **13**, a rigid longitudinal length **14b** extending from head portion **13**, and a distal end **14c**. The distal ends **14c** of the tines terminate in rounded soft flexible tip sections **15**. Lens assemblies **16** are interposed between longitudinal lengths **14b** and flexible tip sections **15**.

In use, the device **10** is applied to an area of scalp **17**, with the flexible tip sections **15** applied to the surface of the scalp **18**. A system of fibre optics **19** channels light from a light source (not shown) through the handle portion **12**, into the head portion **13**, and down through each of the tines **14** towards lens assemblies **16**. Light incident upon lens assemblies **16** is spread by lens assemblies **16** in an outward direction from the distal ends **14c** of tines **14** onto the surface of the scalp **18**. The light emerging from lens assemblies **16** (shown as **21** on figure 3) produces an array of circular light beam footprints (shown as **20** on figure 2) on the surface **18**.

## Claims

1. A hand held hair treatment device (10) for the stimulation of hair growth on the scalp, which device comprises:
a housing (11) which includes a handle portion (12) and a head portion (13);
a plurality of hollow tines (14), each tine having a proximal end affixed to the head portion (13), a longitudinal length (14b) extending from the head portion (13) and a distal end (14c) terminating in a tip section (15);
a light source disposed within the housing and configured to output light;
light guide means (19) for channelling the light from the light source through the tines (14) and along a light guide means axis, and
optical devices (16) which are located at the distal ends of the tines (14);
the optical devices (16) being configured to vector light which is incident upon them transversely to the general light guide means axis;
**characterised in that** the light source is a low power laser having a wavelength ranging from about 600nm to about 1000nm, and an intensity ranging from about 1mW/cm² to about 150mW/cm², a light emitting diode having a wavelength ranging from about 620 to 690 nm, and an intensity ranging from about 1mW/cm² to about 150mW/cm², or a combination thereof and **in that** the optical devices (16) are disposed from 2 to 10 mm from the distal tip of the tine (14) and wherein the tines (14) comprise a longitudinal length (14b) of substantially rigid material, an optical device (16) and a flexible tip portion (15).

2. A device according to claim 1, in which the light guide means (19) is constituted by a system of optical fibres which extends from the light source or sources, into the tines and through the tines.

3. A device according to any of claims 1 or 2 wherein the optical modifier is selected from a lens, optical fibre, mirror, transparent material, semi-transparent material, prism, reflective coating, reflecting groove, beam splitter, light channel or grating.

4. A device according to any one of claims 1 to 3 wherein the optical device is a lens.

5. A device according to any one of claims 1 to 4, in which the optical devices are configured so that they produce an array of circular light beam footprints on the target scalp surface.

## Patentansprüche

1. Tragbare Haarbehandlungsvorrichtung (10) zum Stimulieren von Haarwuchs auf der Kopfhaut, wobei die Vorrichtung umfasst:
ein Gehäuse (11), das einen Griffabschnitt (12) und einen Kopfabschnitt (13) enthält;
mehrere hohle Zacken (14), wobei jeder Zacken ein proximales Ende, das an dem Kopfabschnitt (13) befestigt ist, eine longitudinale Länge (14b), die sich von dem Kopfabschnitt erstreckt (13), und ein distales Ende (14c), das in einem Spitzenabschnitt (15) endet, besitzt;
eine Lichtquelle, die in dem Gehäuse angeordnet ist und konfiguriert ist, um Licht auszugeben;
Licht leitende Mittel (19), um das Licht der Lichtquelle durch die Zacken (14) und entlang einer Achse der Licht leitenden Mittel zu kanalisieren, und optische Vorrichtungen (16), die sich an den distalen Enden der Zacken (14) befinden;
wobei die optischen Vorrichtungen (16) konfiguriert sind, um das Licht, das auf sie einfällt, schräg zu der allgemeinen Achse der Licht leitenden Mittel zu übertragen;
**dadurch gekennzeichnet, dass** die Lichtquelle ein Laser mit geringer Leistung, der eine Wellenlänge in einem Bereich von ungefähr 600 nm bis ungefähr 1000 nm und eine Intensität in einem Bereich von ungefähr 1 mW/cm² bis ungefähr 150 mW/cm² aufweist, eine Leuchtdiode, die eine Wellenlänge in einem Bereich von ungefähr 620 bis 690 nm und eine Intensität in einem Bereich von ungefähr 1 mW/cm² bis ungefähr 150 mW/cm² aufweist, oder
eine Kombination davon ist und dass die optischen Vorrichtungen (16) 2 bis 10 mm von der distalen Spitze des Zackens (14) angeordnet sind, wobei die Zacken (14) eine longitudinale Länge (14b) aus einem im Wesentlichen steifen Material, eine optische Vorrichtung (16) und einen flexiblen Spitzenabschnitt (15) enthalten.

2. Vorrichtung nach Anspruch 1, in der die Licht leitenden Mittel (19) aus einem System aus optischen Fasern, die sich von der Lichtquelle oder den Lichtquellen in die Zacken und durch die Zacken hindurch erstrecken, gebildet sind.

3. Vorrichtung nach einem der Ansprüche 1 oder 2, wobei der optische Modifizierer aus einer Linse, einer optischen Faser, einem Spiegel, einem transparenten Material, einem halb-transparenten Material, einem Prisma, einer reflektierenden Beschichtung, einer reflektierenden Furche, einem Strahlteiler, einem Lichtkanal oder einem Gitter ausgewählt ist.

4. Vorrichtung nach einem der Ansprüche 1 bis 3, wobei die optische Vorrichtung eine Linse ist.

5. Vorrichtung nach einem der Ansprüche 1 bis 4, in der die optischen Vorrichtungen so konfiguriert sind, dass sie eine Anordnung von kreisförmigen Lichtstrahlgrundflächen auf der Zielfläche der Kopfhaut erzeugen.

## Revendications

1. Dispositif à main (10) de traitement capillaire permettant de stimuler la pousse des cheveux sur le cuir chevelu, ledit dispositif comprenant :
un boîtier (11) qui comprend une partie formant manche (12) et une partie formant tête (13) ;
une pluralité de dents creuses (14), chaque dent ayant une extrémité proximale fixée sur la partie formant tête (13), une longueur longitudinale (14b) s'étendant depuis la partie formant tête (13) et une extrémité distale (14c) se terminant en une section formant pointe (15) ;
une source lumineuse disposée à l'intérieur du boîtier et configurée pour émettre de la lumière ;
des moyens de guidage de lumière (19) pour guider la lumière depuis la source lumineuse jusque dans les dents (14) et le long de l'axe des moyens de guidage de lumière, et
des dispositifs optiques (16) qui sont situés sur les extrémités distales des dents (14) ;
les dispositifs optiques (16) étant configurés pour guider la lumière incidente sur ceux-ci transversalement jusqu'à l'axe général des moyens de guidage de lumière ;
**caractérisé en ce que** la source lumineuse est un laser de faible puissance dont la longueur d'onde va d'environ 600 nm à environ 1000 nm et dont l'intensité va d'environ 1 mW/cm² à environ 150 mW/cm², une diode électroluminescente dont la longueur d'onde va d'environ 620 à 690 nm et dont l'intensité va d'environ 1 mW/cm² à environ 150 mW/cm² ou une combinaison de ceux-ci et **en ce que** les dispositifs optiques (16) sont disposés à 2-10 mm de la pointe distale de la dent (14) et les dents (14) comprenant une longueur longitudinale (14b) de matériau sensiblement rigide, un dispositif optique (16) et une partie (15) formant pointe souple.

2. Dispositif selon la revendication 1, dans lequel les moyens de guidage de lumière (19) sont constitués d'un système de fibres optiques qui s'étend depuis la ou les sources lumineuses jusque dans les dents et à travers les dents.

3. Dispositif selon l'une quelconque des revendications 1 ou 2, dans lequel le modificateur optique est choisi parmi une lentille, une fibre optique, un miroir, un matériau transparent, un matériau semi-transparent, un prisme, un revêtement réfléchissant, une rainure réfléchissante, un séparateur de faisceau, un canal lumineux ou une grille.

4. Dispositif selon l'une quelconque des revendications 1 à 3, dans lequel le dispositif optique est une lentille.

5. Dispositif selon l'une quelconque des revendications 1 à 4, dans lequel les dispositifs optiques sont configurés de manière à produire un ensemble d'empreintes circulaires par faisceau lumineux sur la surface ciblée du cuir chevelu.
